Europäisches Patentamt

European Patent Office

Office européen des brevets

(11) Publication number: **0 153 831 B1**

(12) **EUROPEAN PATENT SPECIFICATION**

(45) Date of publication of patent specification: **02.05.91**    (51) Int. Cl.⁵: **A61F 2/08**, A61B 17/16

(21) Application number: **85300931.4**

(22) Date of filing: **13.02.85**

(54) **Instruments for use in the surgical replacement of ligaments.**

(30) Priority: **13.02.84 EP 84300875**

(43) Date of publication of application:
**04.09.85 Bulletin  85/36**

(45) Publication of the grant of the patent:
**02.05.91 Bulletin  91/18**

(84) Designated Contracting States:
**AT BE CH DE FR GB IT LI LU NL SE**

(56) References cited:
**EP-A- 0 126 520**
**BE-A- 531 833**
**US-A- 3 913 566**
**US-A- 4 059 115**
**US-A- 4 096 749**

(73) Proprietor: **Seedhom, Bahaa Botros**
**75 Holt Park Crescent**
**Leeds 16 West Yorkshire(GB)**

Proprietor: **Fujikawa, Kyosuke**
**Department of Orthopaedic Surgery Keio**
**School of Medicine**
**Tokyo(JP)**

(72) Inventor: **Seedhom, Bahaa Botros**
**75 Holt Park Crescent**
**Leeds 16 West Yorkshire(GB)**
Inventor: **Fujikawa, Kyosuke**
**Department of Orthopaedic Surgery Keio**
**School of Medicine**
**Tokyo(JP)**

(74) Representative: **Orr, William McLean et al**
**URQUHART-DYKES & LORD 5th Floor, Tower**
**House Merrion Way**
**Leeds West Yorkshire, LS2 8PA(GB)**

## Description

This invention relates to surgical instruments for use in the surgical replacement of a ligament and in particular for implanting a prosthetic ligament into the body.

The present invention is concerned with a method of securing a prosthetic ligament to a host bone comprising the steps of:

removing a cylindrical bone plug from one surface of the host bone to leave a cylindrical socket in the host bone;

forming a bore of smaller diameter than the cylindrical socket coaxial with the socket extending from the cylindrical socket to an opposing surface of the host bone;

drawing the prosthetic ligament through the cylindrical socket and the bore;

replacing the bone plug into the socket thereby to capture the prosthetic ligament between the bone plug and the host bone.

The present invention is also concerned with a method of securing a prosthetic ligament to a first and second host bone to extend between a first surface of the first host bone and a second surface of the second host bone comprising the steps of:

removing a first cylindrical bone plug from a third surface opposite to the first surface of the first host bone plug, to leave a first cylindrical socket;

forming a first bore of smaller diameter than the first socket, coaxial with the first socket and extending from the socket to the first surface of the first host bone;

removing a second cylindrical bone plug from a fourth surface opposite to the second surface of the second host bone to leave a second host socket;

forming a second bore of smaller diameter than the second socket coaxial with the second socket and extending from the socket to the second surface of the second host bone to form a shoulder between the socket and the bore;

providing a prosthetic ligament including at one end a pouch portion capable of receiving a cylindrical bone plug and including an opening through which a bone plug may pass into the pouch portion;

passing the prosthetic ligament thorough the first socket, the first bore, the second bore and the second socket, whereby said pouch portion lies beyond said second socket;

inserting the second bore plug through the opening to lie within the pouch portion;

The present invention is further concerned with a technique for cutting and removing a replaceable bone plug from a host bone comprising:

forming an annular channel in the host bone to define the periphery of the plug;

applying a sharp blow to the plug along a direction transverse to the axis of the plug to sheat the plug from the host bone; and

removing the sheared plug from the host bone.

Preferably, before applying the sharp blow, the plug is gripped by an instrument about an arcuate portion of its circumference and fully along its length, said arcuate portion being no greater than about 330°, and the sharp blow is applied to the instrument along a direction which is toward the ungripped portion of the plug.

Prosthetic ligament and instruments for use in the surgical replacement of ligaments is described in European Patent No. 0126520 and forms part of the state of the art according to Art54(2)EPC due to Art87(4)EPC.

The invention described therein relates to a prosthetic ligament and a set of surgical instruments for use in the surgical replacement of a ligament into the body. The ligament comprises an elongate foraminous flexible strip, the size and distribution of the perforations being substantially uniform along the length of the ligament.

According to this invention a tool for extracting a cylindrical bone plug from a bone in which an annular channel has been formed to define the periphery of said bone plug, said tool being adapted to sever the base of said plug from the bone, the tool comprising:

a grippable handle portion at one end;

a wall portion at the other end, the wall portion defining a hollow, open-ended cylinder adapted to receive the bone plug;

a longitudinally extending slot formed in the cylindrical wall;

said wall being adapted to be received within said annular channel of said bone with the bone plug being receivable within the open-ended cylinder, said slot defining an arcuate void region of the annular space in the bone, and further comprising indicia means on the tool indicating that region of the tool which is diametrically opposed to the longitudual slot.

The bone plug is extracted by inserting the cylindrical wall into the annulus. A short, sharp blow is applied in the middle region of the extractor while the handle is gripped, the force being applied at a point in said middle region but on the side opposite to the slot. This severs the bone plug completely from the host bone.

The indicia means indicates the correct area to be struck. More preferably this comprises a flattened bearing surface.

The bone plug extractor includes the grippable handle which must be gripped whilst the force is applied so that no bone damage occurs.

The bone plug extractor preferably includes means to aid turning the extractor about its longitudinal axis. For instance, the handle may include gripping means such as flutes or a knurled area. In addition the handle may include a hole or holes through which a torque bar may be introduced to extend transversely of the handle and by means of which additional torque may be exerted should the friction between the extractor and the bone plug be too high to be overcome by merely gripping the handle.

When the bone plug has been removed a cylindrical drill bit guide is placed into the cylindrical socket formed in the bone. The drill bit guide has a through hole for accommodating a drill bit which is introduced into the guide so that a reduced diameter bore is then drilled from the base of the cylinder to the other side of the bone at a region where the single pin of the clamp engages the bone. This region is selected to be as close as possible to the position where the original ligament was attached to the bone.

This process is then repeated with the other bone to which the ligament is to be attached, the same instruments being used again.

A ligament of the type with which this invention is concerned may be used to replace the cruciate ligaments of the knee. It can also, in the form of, for instance, a flat strip, be used to replace the medial ligament of the knee, or other ligaments or tendons of other joints.

A bone plug extractor tool according to the invention will now be described by way of example only with reference to the accompanying drawings, in which:

Figure 1 is a perspective view of a prosthetic ligament;

Figure 2 is a perspective view of a set of surgical instruments incorporating the bone plug extractor.

Figure 4 is a perspective view of the bone plug extractor;

Figure 4A is a view on that end of the bone plug extractor remote from the handle portion;

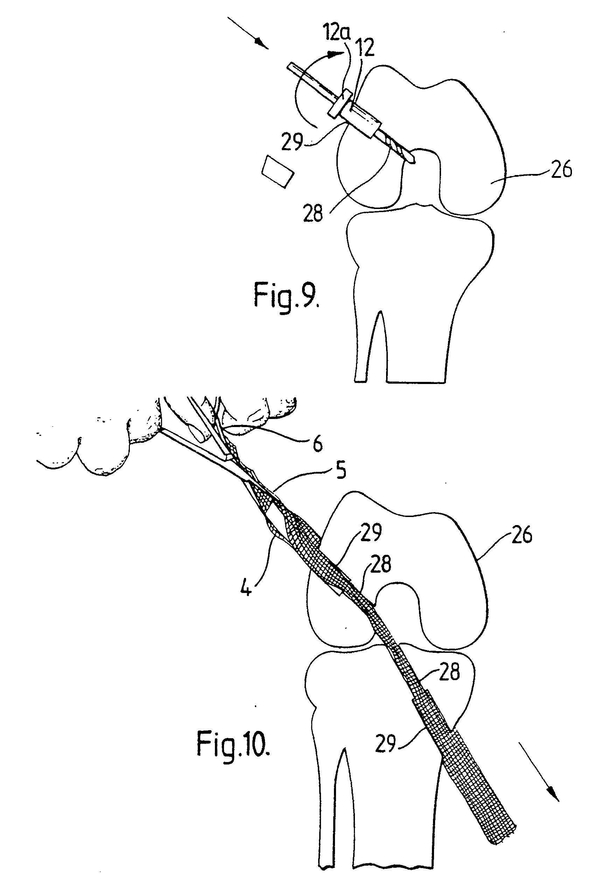

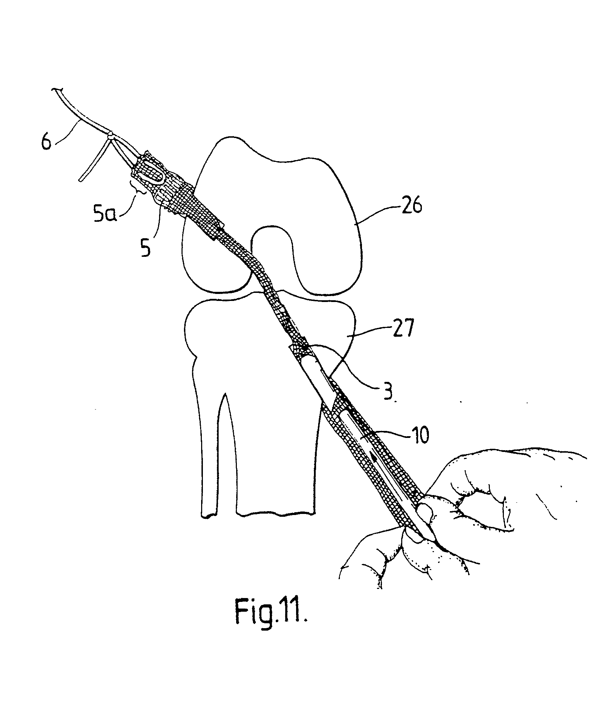

Figures 5 to 11 are representations (schematic only) of the steps of a method of replacing an anterior cruciate ligament in the knee.

An artificial ligament 1 comprises an elongate strip of open weave polyester. This fabric is Terylene and is a mock leno weave comprising a warp of 550 Decitex polyester yarn and a weft of twisted polyester yarn. The holes are 0.1 x 0.2 cm. There are fourteen to fifteen holes across the width of the strip, and twenty holes per cm². 

The ligament is in the form of a tube 2 which has a slit 3 at one open end of the ligament 1 which extends to the open end edge of the ligament and a slit 4 at the other closed end of the ligament. The end of the slit 4 remote from the centre of the tube 2 is closed by a row or more of stitching or by dense weaving or in some other manner, to form a pouch capable of receiving a bone plug. As far as at the end of this row of stitching or densely woven section, the ligament extends further by a short portion 5a of the same open weave as the central portion 2. A threading cord 6 is attached to a position on this shorter portion 5a from the closure portion. The purpose of threading cord 6 is to allow the ligament to be pulled through the bores in the bone.

A bone plug may be inserted into the pouch through the slit 4. The stitching or densely woven section 5 forms a closure which prevents movement of the bone plug out of the pouch in a direction away from the centre of the ligament 1.

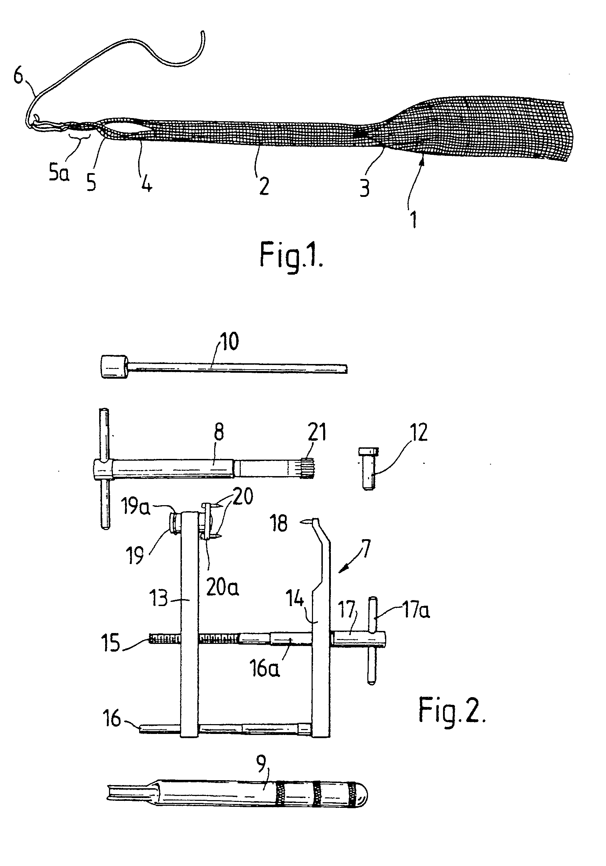

In order to illustrate the slit 4, the ligament 1 has been shown in Figure 1 with a light coloured tubular insert. This means that the pouch-like structure is not clearly illustrated.

A set of surgical instruments for use in the implantation of the ligament comprises a clamp 7, a reamer 8, a bone plug extractor 9, a push rod 10 and a drill bit guide 12.

Clamp 7 comprises two parallel limbs 13 and 14 interconnected by a main spacer rod 15 and an auxililary rod 16. Main spacer rod 15, approximately centrally located in the clamp, is threaded from one end portion to a position lying about one third to one half of the length of the rod from that end portion. The threaded portion is in threaded engagement with a threaded bore located in limb 13 at the appropriate distances from the ends. The remainder of rod 15 is unthreaded and extends from the threaded portion through limb 14 to terminate in an enlarged head portion 17 carrying a handle 17a. For much of its length, this unthreaded portion of rod lies within a sleeve 16a which extends through limb 14 to which it is fixed. Within limb 14 sleeve 16a defines the bore through which rod 15 extends.

Auxiliary rod 16 is fixed at one end to limb 14 and extends through a bore or a slot in the other limb 13 for sliding movement therewithin.

The limb 14 includes a guide pin 18 extending perpendicularly from that end of the limb 14 remote from auxiliary rod 16. In use guide pin 18 engages the bone to locate the position of attachment of the ligament to the bone.

The other limb 13 has mounted on it a cylindrical guide 19 which is rotatably mounted within a bore in limb 13 and is provided with a circlip 19a to prevent movement of the guide out of its bore in limb 13. The other end of cylindrical guide 13 is in the form of an enlarged section or pin support 20a which in turn carries two pins 20. Extending through cylindrical guide 19 is a bore for receiving the reamer 8 such that reamer 8 will extend in a direction towards guide pin 18. The axes of guide pin 18 and said cylindrical guide are collinear.

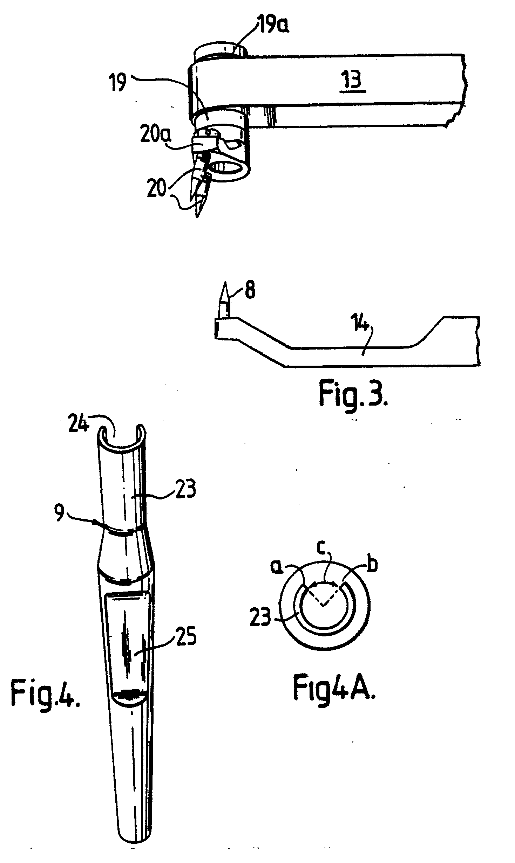

As best seen in Figure 3, pins 20 are mounted on support 20a in a position offset from the bore extending through the guide 20 and at an angle to the axis of this bore. Guide 19 may be rotated within limb 13 so that the pins 20 describe the circumferential movement about the centre of the bore extending through guide 19. The position and orientation of the pins on guide 19 take account of the different contours of bone with which the clamp 7 may be used.

The reamer 8 passes through the cylindrical guide 19 and has sharp cutting edges 21, which are milled along its surfaces and form sharp teeth at the rim of the reamer, and a handle 22 which may be used to oscillate the cutting edges 21 to remove the bone dust.

The bone plug extractor 9 includes at one end a cylindrical wall 23 which defines an open ended cylinder. At its other end the tool 9 includes a grippable handle 23a.

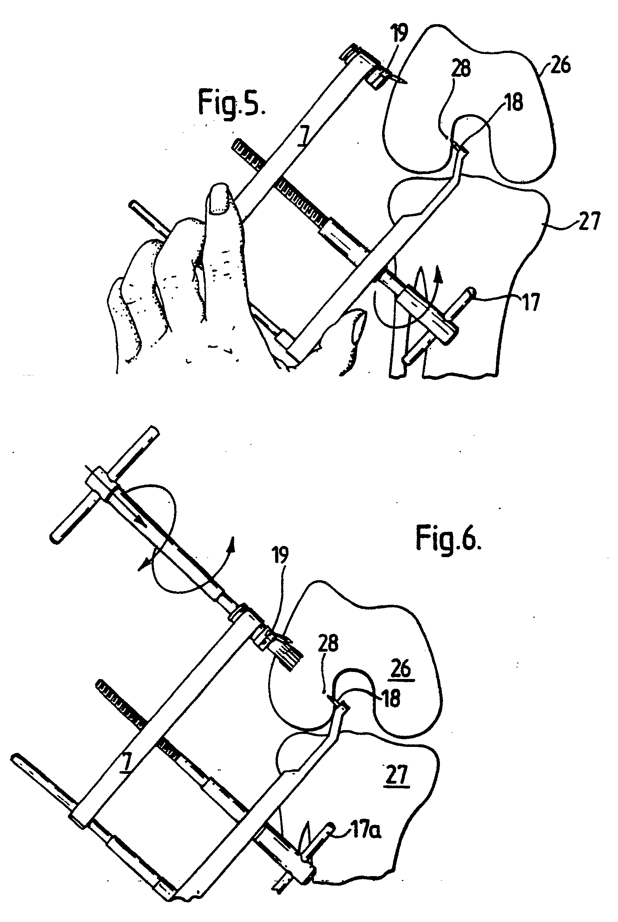

Extending from the edge of the wall 23 is a slot 24 which defines an arcuate void in the cylindrical wall of the same length as that of section 23. The angle circumscribed by the slot is about 80°. As shown in Figure 4A, this angle c is subtended between the ends a, b of the slot and the centre of the circular portion of the wall 23.

On the opposite side of the bone plug extractor 9 to the slot 24 is a flattened bearing surface 25. This defines an area to be struck in order to sever a base of a bone plug being removed by the extractor tool 9.

The drill bit guide 12 is placed in the cylindrical hole created in the bone and is used as a guide for the drill bit to drill out the reduced diameter portion of the bore.

The anterior cruciate ligament in the knee is connected to the femur 26 and the tibia 27. As showin in Figure 5, the clamp 7 is placed around the femur 26 with the first guide pin 18 placed at a point within the region of attachment 28 where the original ligament was attached to the femur 26. The clamp is then rotated until the cylindrical guide 19 is at the opposite side of the femur 26. After adjusting the cylindrical guide such that the two pins engage the bone, the clamp 7 is then tightened up using handle 17a to locate the axis of the bore.

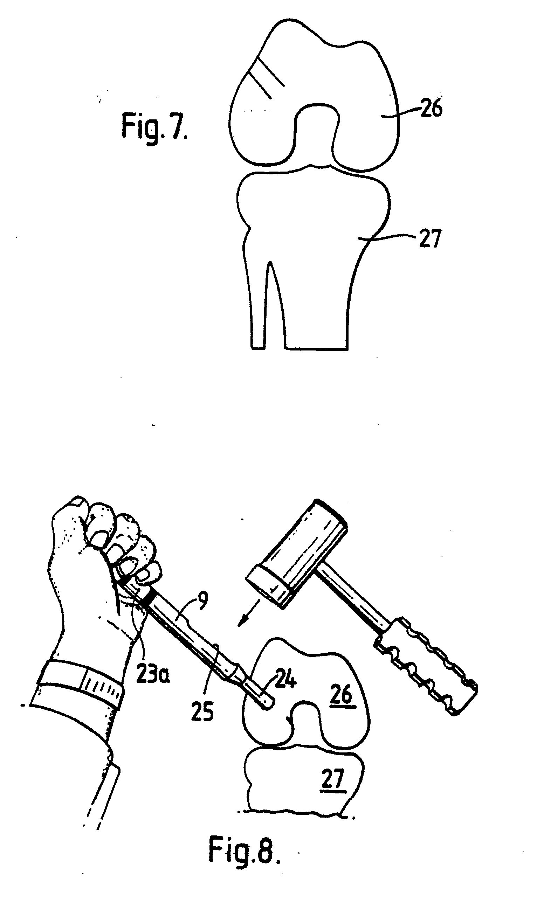

As shown in Figure 6, the reamer 8 is then placed within the cylindrical guide 19 and the handle 22 is oscillated whilst applying a force along the axis of the reamer to remove an annulus of bone dust (Figure 7). The reamer is then removed from the bone. In the infrequent possibility that the bone plug is removed within the reamer, the push rod 10 is inserted into the reamer to push the bone plug thereout.

As shown in Figure 8, the bone plug extractor 9 is then placed in the annulus defined in the femur 26. The gripping area 23a is held firmly to secure the bone plug extractor 9 firmly in place. A swift force is applied to the flattened surface 25 to remove the bone plug from the bone, by severing the base of the bone plug from the femur 26. The bone plug may be removed from the bone plug extractor 9 by inserting a rod into opening 24.

The drill bit guide 12 is then placed in the cylindrical hole left in the femur 26. The enlarged end 12a of drill bit guide 12 prevents the guide being pushed too far into the hole in the femur so that it is subsequently difficult to extract. A drill bit is then placed within the guide 12 to drill out the reduced diameter portion 28 of the bore 29 now formed through the femur 26.

The procedure is then repeated in the tibia forming a similar bore in the tibia. The ligament 1 is then threaded through the two bores 29 using the cord 6. The bone plug from the femur 26 is then placed within the slit 4 in the ligament.

The other end of the ligament 1 is then pulled forcing the bone plug against the shoulder formed between the enlarged portion and the reduced diameter portion 28 by the closed end 5.

The bone plug from the tibia 27 is then forced into the opening 3, aided by a push rod. Thus the ligament 1 is secured by bone plugs within both the femur and tibia.

The ligament is stapled to the tibia and is then folded on itself. It is then stitched to itself and the periosteal tissue. The remainder of the ligament is then cut off.

At the end which emerges from the femur, the ligament is sutured to the periosteal tissue and the cord is clipped off. The remaining part of the ligament may be trimmed but at least some distance away from the closed end of the patch defined by the densely woven region.

Once the ligament is implanted within a patient, the ligament will be subject to tissue ingrowth thereby strengthening the ligament and also the anchoring of the ligament to the bones.

## Claims

1. A tool for extracting a cylindrical bone plug from a bone in which an annular channel has been formed to define the periphery of said bone plug, said tool being adapted to sever the base of said plug from the bone to enable complete separation of the plug from the bone, the tool comprising:
a grippable handle portion at one end;
a wall portion at the other end, the wall portion defining a hollow, open-ended cylinder adapted to receive the bone plug;

a longitudinally extending slot formed in the cylindrical wall;

said wall being adapted to be received within said annular channel of said bone with the bone plug being receivable within the open-ended cylinder, said slot defining an arcuate void region of the annular space in the bone and further comprising;

indicia means on the tool indicating that region of the tool which is diametrically opposed to the longitudinal slot.

2. A bone plug extractor tool as defined in claim 1 wherein the indicia means comprises a flattened bearing surface, said flattened bearing surface defining a strikable surface.

3. A bone plug extractor tool as defined in claim 1 or claim 2 wherein the angle circumscribed by said slot is substantially between 30° and less than 180°.

4. A bone plug extractor tool as defined in any of the preceding claims, wherein the angle circumscribed by said slot is substantially from 70° to 90°.

**Revendications**

1. Outil d'extraction d'une cheville cylindrique d'os à partir d'un os dans lequel a été formé un canal annulaire pour définir la périphérie de la cheville d'os, l'outil étant adapté pour séparer la base de la cheville de l'os pour permettre une séparation complète de la cheville de l'os, l'outil comprenant :
   - une partie formant poignée de préhension à une extrémité ;
   - une partie formant paroi à l'autre extrémité, la partie formant paroi définissant un cylindre creux, à extrémité ouverte, adapté pour recevoir la cheville d'os ;
   - une fente s'étendant longitudinalement formée dans la paroi cylindrique ; ladite paroi étant adaptée pour être reçue dans ledit canal annulaire de l'os, la cheville d'os pouvant être reçue dans le cylindre à extrémité ouverte, la fente définissant une région vide, en arc de cercle, de l'espace annulaire de l'os et l'outil comprenant en outre des moyens indicateurs indiquant quelle est la partie de l'outil diamétralement opposée à la fente longitudinale.

2. Outil extracteur de cheville d'os selon la revendication 1, caractérisé en ce que les moyens indicateurs comportent une surface portante plate, la surface portante plate définissant une surface pouvant être frappée.

3. Outil extracteur de cheville d'os selon la revendication 1 ou 2, caractérisé en ce que l'angle circonscrit par la fente est sensiblement compris entre 30 degrés et 180 degrés.

4. Outil extracteur de cheville d'os selon l'une quelconque des revendications précédentes, caractérisé en ce que l'angle circonscrit par la fente est sensiblement de 70 à 90 degrés.

**Ansprüche**

1. Werkzeug zum Herausholen eines zylindrischen Knochenpfropfens aus einem Knochen, in dem ein ringförmiger Kanal ausgebildet worden ist, um den Umfang des Knochenpfropfens zu definieren, wobei das Werkzeug zum Abtrennen der Basis des Pfropfens vom Knochen ausgebildet ist, um eine völlige Trennung des Pfropfens vom Knochen zu ermöglichen, und wobei das Werkzeug folgende Bestandteile umfaßt:

   Einen ergreifbaren Griffteil an seinem einen Ende, einen Wandteil am anderen Ende, wobei der Wandteil einen hohlen, am Ende offenen Zylinder umschließt, der dazu dient, den Knochenpfropfen aufzunehmen, einen in der zylindrischen Wand ausgebildeten, sich in Längsrichtung erstreckenden Schlitz,

   wobei die Wand ausgebildet ist, um in dem ringförmigen Kanal des Knochens aufgenommen zu werden, während der Knochenpfropfen in dem am Ende offenen Zylinder aufnehmbar ist, und wobei der Schlitz einen gebogenen Leerraum des ringförmigen Raums im Knochen umgrenzt, und wobei das Werkzeug weiterhin auf dem Werkzeug angebrachte Anzeigemittel umfaßt, die den Bereich des Werkzeugs kennzeichnen, der dem in Längsrichtung verlaufenden Schlitz diametral gegenüberliegt.

2. Werkzeug zum Herausholen eines Knochenpfropfens nach Anspruch 1, bei dem die Anzeigemittel eine abgeflachte Auflagefläche umfassen, wobei die abgeflachte Auflagefläche eine Schlagfläche definiert.

3. Werkzeug zum Herausholen eines Knochenpfropfens nach Anspruch 1 oder 2, bei dem der von dem Schlitz umschlossene Winkel im wesentlichen zwischen 30° und weniger als 180° liegt.

4.  Werkzeug zum Herausholen eines Knochenpfropfens nach einem der vorhergehenden Ansprüche, bei dem der vom Schlitz umschlossene Winkel im wesentlichen zwischen 70° und 90° liegt.

Fig.1.

Fig.2.

Fig.3.

Fig.4.

Fig4A.

Fig.5.

Fig.6.

Fig. 7.

26

27

9

23a

25

24

26

27

Fig.8.

Fig.9.

Fig.10.

Fig.11.